# EUROPÄISCHE PATENTSCHRIFT

(11) **EP 1 535 642 B1**
(45) Veröffentlichungstag und Bekanntmachung des Hinweises auf die Patenterteilung: **26.08.2015**
(21) Anmeldenummer: 04028056.2
(22) Anmeldetag: 25.11.2004
(51) Int. Cl.: A61M 16/04, F16B 2/08

(54) **Beissring**
Bite block
Anneau de protection dentaire

(30) Priorität: 25.11.2003 DE 20318238 U
(43) Veröffentlichungstag der Anmeldung: 01.06.2005
(73) Patentinhaber: Maslanka, Herbert, 78532 Tuttlingen (DE)
(72) Erfinder: Maslanka, Herbert, 78532 Tuttlingen (DE)
(74) Vertreter: Weickmann & Weickmann

(56) Entgegenhaltungen:
- FR-A- 2 664 817
- GB-A- 1 133 159
- GB-A- 1 135 102
- US-A- 3 774 616
- US-A- 4 502 478
- US-A- 5 174 284
- US-A- 5 305 742
- US-A1- 2002 151 871

## Beschreibung

Die vorliegende Erfindung betrifft einen Beißring mit einem eine Durchlassöffnung zum Einführen eines Instruments in den Mund einer Person umschließenden Beißtubus und einem von auf sich gegenüberliegenden Seiten einer Mittelebene des Beißtubus von diesem quer zur Mittelebene abstehenden Lippenschild.

Beißringe dieser Art werden im medizinischen Bereich bei der Behandlung oder Untersuchung von Patienten eingesetzt, wenn ein Instrument durch den Mund des Patienten in den Körper eingeführt werden soll. Ein solches Instrument kann beispielsweise ein Endoskop, ein Versorgungsschlauch, ein Operationsinstrument oder dergleichen sein. Um den Mund zum Einführen des Instruments und während des Untersuchungs- bzw. Behandlungsvorgangs in einer geeigneten geöffneten Stellung zu halten und eine Verletzung des eingeführten Instruments durch die Zähne des Patienten zu verhindern, wird der Beißtubus des Beißrings in den Mund des Patienten eingeführt, bis das Lippenschild auf den Ober- bzw. Unterlippen des Patienten anliegen. Der Patient, der auf das oftmals unangenehme Einführen des Instruments mit einer bewussten oder unbewussten Abwehrreaktion reagiert und dabei zum Beispiel versucht, den Mund zu schließen, wird durch den Beißring daran gehindert, in das Instrument zu beißen.

Die bekannten Beißringe haben den Nachteil, dass sie nur unzureichend an die Anatomie verschiedener Patienten angepasst sind. So kann beispielsweise ein Beißring, dessen oberer Lippenschildbereich bei einer Person mit natürlichem Gebiss die Oberlippe zurückhält und unterhalb der Nase bequem anliegt, bei einer Person, deren künstliches Gebiss vor dem Eingriff entfernt wurde, sehr unbequem anliegen, da der dann höher liegende obere

Lippenschildbereich an der Unterseite der Nase anstößt. Andererseits ist es möglich, dass ein Beißring, dessen oberer Lippenschildbereich bei einem Patienten ohne Gebiss ausreichend hoch ist, um die Oberlippe zuverlässig zurückzuhalten, nicht für einen Patienten mit Gebiss geeignet ist, da sich die Oberlippe eines solchen Patienten dann möglicherweise über den oberen Lippenschildbereich schieben könnte. Bislang ist es daher notwendig, in der betreffenden medizinischen Einrichtung mindestens zwei verschiedene Typen von Beißringen vorrätig zu halten, einen Typ mit relativ hohem oberen Lippenschildbereich für Patienten mit natürlichem Gebiss und einen mit relativ niedrigem oberen Lippenschildbereich für Patienten ohne Gebiss. Dies verursacht erhöhten Organisations- und Kostenaufwand.

Die US 5,174,284 A offenbart einen Beißring mit einem Beißtubus sowie einem allgemein senkrecht von diesem abstehenden Lippenschild. An einem dem Patienten zugewandten Ende des Beißtubus ist am unteren Randabschnitt des Beißtubus ein Zugenniederhalter angeformt, welcher eine Zungenniederhalterfläche aufweist, um im eingesetzten Zustand des Beißrings die Zunge des Patienten nach unten zu drücken, sodass diese das Einführen von Instrumenten nicht behindern kann.

Ferner offenbart die US 5,305,742 A einen Beißring mit einem Lippenschild, einer zentralen Durchlassöffnung und zwei diametral bezüglich der Durchlassöffnung gegenüberliegenden, vom Lippenschild senkrecht abstehenden Beißstegen. An der vom Patienten abgewandten Seite des Lippenschilds weist die Durchlassöffnung Rastausnehmungen auf, an welche ein Verbindungsstück zum Anschluss eines T-Stück-Verbinders in horizontaler Position angeklipst werden kann.

Die US 2002/0151871 A1 offenbart einen Beißring mit den Merkmalen des Oberbegriffs des Anspruchs 1.

Demgegenüber liegt der vorliegenden Erfindung die Aufgabe zugrunde, einen Beißring der oben genannten Art bereitzustellen, welcher auf einfache Weise besser für die Verwendung bei Patienten unterschiedlicher Anatomie angepasst ist. Insbesondere ist es eine Aufgabe der vorliegenden Erfindung, einen Beißring der oben genannten Art bereitzustellen, welcher gleichermaßen für Patienten mit und ohne Gebiss verwendet werden kann.

Zur Lösung dieser Aufgabe stellt die Erfindung einen Beißring nach Anspruch 1 bereit. Demnach ist gemäß der Erfindung vorgesehen, dass der Beißtubus des Beißrings, bezogen auf die Mittelebene des Beißtubus im Wesentlichen symmetrisch ausgebildet ist, der Beißtubus bezogen auf die Mittelebene im Wesentlichen symmetrisch ausgebildet ist und der Lippenschild zumindest an seinem mittig zum Beißtubus gelegenen Bereich auf einer Seite der Mittelebene niedriger ist als auf seiner gegenüberliegenden Seite.

Ein solcher Beißtubus weist dann beiderseits der Mittelebene jeweils einen Lippenschildbereich auf, wobei bezüglich der Mittelebene einer der Lippenschildbereiche niedriger ist als der andere Lippenschildbereich. Da der im Mund des Patienten aufnehmbare Beißtubus bezogen auf die Mittelebene im Wesentlichen symmetrisch ausgebildet ist, ist der erfindungsgemäße

Beißring in einem ersten eingesetzten Zustand verwendbar, in welchem einer der Lippenschildbereiche an der Oberlippe des Patienten und der andere Lippenschildbereich an der Unterlippe des Patienten anliegt, und kann darüber hinaus in einem zweiten eingesetzten Zustand verwendet werden, in welchem der Beißring gegenüber dem ersten eingesetzten Zustand entlang der Achse der Durchlassöffnung um 180° gedreht ist, so dass jeder der Lippenschildbereiche dann jeweils an der anderen Lippe aus Oberlippe und Unterlippe des Patienten anliegt.

Ist der niedrigere Lippenschildbereich gemäß der Anatomie einer Bezugsperson ohne Gebiss ausgebildet und ist der höhere Lippenschildbereich gemäß der Anatomie einer Bezugsperson mit Gebiss ausgebildet, so ist es möglich, für beide Patientengruppen denselben Typ Beißringe zu verwenden, indem der erfindungsgemäße Beißtubus bei einem Patienten mit Gebiss so eingeführt wird, dass der höhere Lippenschildbereich an der Oberlippe des Patienten anliegt, und bei einem Patienten ohne Gebiss der Beißring so eingeführt wird, dass der niedrigere Lippenschildbereich an der Oberlippe des Patienten anliegt. Auf diese Weise können Kosten und Organisationsaufwand eingespart werden, da in der betreffenden medizinischen Einrichtung nur ein Typ von Beißringen vorrätig gehalten werden muss.

Der Beißring, insbesondere der Lippenschild und der Beißtubus des Beißrings, können im Rahmen der Erfindung verschiedene von herkömmlichen Beißringen bekannte Formen oder andere zweckmäßige Einrichtungen aufweisen. In einer Ausführungsform der Erfindung wird vorgeschlagen, dass bezüglich der Mittelebene ein äußerer Rand des niedrigeren Lippenschildbereichs eines konkave Form aufweist und ein äußerer Rand des höheren Lippenschildbereichs eine konvexe Form aufweist. Eine solche Gestaltung des Lippenschilds hat den Vorteil, dass bei der Verwendung des Beißrings bei einem Patienten mit Gebiss der an der Unterlippe anliegende Teil des Lippenschilds auf Grund der konkaven Gestalt beiderseits seines mittig zum Beißtubus gelegenen Lippenschildbereichs jeweils einen Flügelschildbereich aufweist, in welchem die Höhe des Lippenschilds größer ist als in dem mittig zum Beißtubus gelegenen Bereich. Damit ist es trotz des relativ niedrigen mittleren Lippenschildbereichs möglich, die Unterlippe des Patienten insgesamt zuverlässig zurückzuhalten. Ein ähnlicher Effekt ergibt sich, wenn ein solcher Beißring bei einem Patienten ohne Gebiss so eingeführt ist, dass der niedrigere Lippenschildbereich an der Oberlippe anliegt. Unterstützt durch die Flügelschildbereiche hält der konvexe Lippenschildbereich die Oberlippe zuverlässig zurück und bildet dabei eine Aussparung für die Nase des Patienten, um ein unangenehmes Drücken oder Reiben des oberen Rands des Lippenschilds an der Unterseite der Nase zu vermeiden.

Alternativ ist es jedoch auch möglich, den Lippenschild beiderseits der Mittelebene konkav auszubilden oder einen geraden oder anderen Randverlauf zu wählen.

Hinsichtlich der Ausgestaltung des Lippenschilds eines Beißrings gemäß der Erfindung ist vorgesehen, dass die Höhe des niedrigeren Lippenschildbereichs über einer zugewandten Beißfläche des Beißtubus zwischen 2 und 15 mm, vorzugsweise zwischen 6 und 12 mm liegt, und die Höhe des höheren Lippenschildbereichs über einer zugewandten Beißfläche des Beißtubus zwischen 10 und 30 mm, vorzugsweise zwischen 15 und 25 mm liegt. Durch eine derartige Ausgestaltung des Lippenschilds wird für Patienten verschiedener Anatomie einerseits ein angenehmes Anliegen des Beißrings im Mund und andererseits eine zuverlässige Funktion zur Unterstützung der Arbeit des medizinischen Personals sichergestellt.

Auch für die Durchlassöffnung des Beißtubus sind verschiedenste Ausgestaltungsmöglichkeiten denkbar. So wird etwa daran gedacht, dass die Durchlassöffnung des Beißtubus zumindest im Bereich des Lippenschilds im Wesentlichen die Querschnittsform eines Rechtecks, insbesondere eines Rechtecks, dessen längere Seiten parallel zur Mittelebene liegen, aufweist.

Dies kann von Vorteil sein, um eine Führung für ein entsprechend geformtes Instrument bereitzustellen.

Nach einer weiteren Ausführungsform stellt die vorliegende Erfindung auch einen Beißring der vorstehend beschriebenen Art mit einem an diesem angebrachten oder anbringbaren oder ausgebildeten Halteband bereit. Im Stand der Technik finden sich Haltebänder, welche aus einem elastischen Material hergestellt sind und entlang ihrer Längsrichtung eine Mehrzahl von Löchern aufweisen, welche wahlweise in Einhängeabschnitte des Beißrings einhängbar sind, um die wirksame Länge des Haltebandes dem Kopfumfang des Patienten anzupassen. Bei den herkömmlichen Haltebändern tritt das Problem auf, dass die Festigkeit des Haltebands in den Bereichen der Löcher geringer ist und die Haltebänder beim Gebrauch dazu neigen an diesen Stellen zu reißen. Um dieser Gefahr zu begegnen muss die Gesamtbreite bzw. Materialstärke der Haltebänder erhöht werden, was zu erhöhtem Materialaufwand führt.

Um diese Nachteile zu vermeiden und bei möglichst geringem Materialverbrauch ein stabiles elastisches Halteband bereitzustellen, schlägt die vorliegende Erfindung vor, entlang des Haltebandes eine Mehrzahl von durch Löcher oder Ausnehmungen in dem Halteband gebildete Anbringungsabschnitte anzuordnen, welche an mindestens einem Einhängeabschnitt des Beißrings einhängbar sind, wobei die Breite oder/und die Dicke des Haltebandes in den Anbringungsabschnitten größer ist als in den Anbringungsabschnitten benachbarten Abschnitten des Haltebandes. Auf diese Weise kann die Schwächung des Haltebandes in den Anbringungsabschnitten aufgrund der dort eingebrachten Löcher oder Ausnehmungen durch eine Verbreiterung oder/und Verdickung des Haltebandes in diesen Abschnitten ausgeglichen werden, so dass das Halteband insgesamt eine ausreichende Reißfestigkeit bei vergleichsweise geringem Materialverbrauch aufweist. Anders herum betrachtet spart die Erfindung Haltebandmaterial nur in den Bereichen ein, in denen keine Löcher oder Ausnehmungen vorgesehen sind, so dass eine Verringerung der Reißfestigkeit vermieden werden kann.

Um die beiden gegenläufigen Einflüsse, Materialeinsparung und Reißfestigkeit, besser aufeinander abzustimmen, können die Breite oder/und die Dicke des Haltebandes in den Anbringungsabschnitten und die Breite oder/und die Dicke des Haltebandes in den den Anbringungsabschnitten benachbarten Abschnitten des Haltebandes so gewählt sein, dass für eine Zugbeanspruchung entlang der Längsrichtung des Haltebandes eine Reißfestigkeit des Haltebandes in allen in der Längsrichtung aufeinanderfolgenden Abschnitten des Haltebandes im Wesentlichen gleich groß ist. Für eine vorgegebene Mindestreißfestigkeit kann somit ein Halteband mit annähernd minimalem Materialverbrauch bereitgestellt werden.

Um eine gute Verstellbarkeit des Haltebandes zu gewährleisten, können entlang des Haltebandes eine Mehrzahl von Anbringungsabschnitten angeordnet sein. Das erfindungsgemäße Halteband kann dann vorteilhaft so ausgestaltet sein, dass die beiden Längsränder des Haltebandes zumindest abschnittsweise in einer Hauptebene des Haltebandes derart wellenförmig verlaufen, dass Abschnitte mit größerer Breite und Abschnitte mit kleinerer Breite abwechselnd ineinander übergehen, wobei die Anbringungsabschnitte in den Abschnitten größerer Breite vorgesehen sind.

Als Materialien für das Halteband kommen insbesondere thermoplastische Elastomere (TPE) in Betracht. Von Vorteil sind vor allem latexfreie Materialien. Thermoplastische Elastomere weisen eine hohe Dehnbarkeit und eine gute Reißfestigkeit auf und sind im Wesentlichen geruchsfrei. Für die Herstellung eines Haltebandes ist insbesondere ein Spritzgussverfahren von Vorteil. Um die Materialstruktur, insbesondere im Hinblick auf die Reiß-festigkeit, im Bereich der Löcher oder Ausnehmungen zu verbessern, ist es ferner von Vorteil, die Löcher oder Ausnehmungen ebenfalls bereits wäh - rend der Formung des Haltebands in dem Spritzgussverfahren mit auszubilden.

Die Erfindung wird im Folgenden anhand einer Ausführungsform unter Bezugnahme auf die beigefügten Zeichnungen näher erläutert. Es zeigen
- Figur 1: eine Vorderansicht eines Beißrings gemäß einer Ausführungsform der vorliegenden Erfindung,
- Figur 2: eine Rückansicht des Beißrings,
- Figur 3: eine perspektivische Rückansicht des Beißrings,
- Figur 4: eine Seitenansicht des Beißrings und
- Figur 5: eine Draufsicht auf ein Ausführungsbeispiel eines erfindungs-gemäßen Haltebands für einen Beißring.

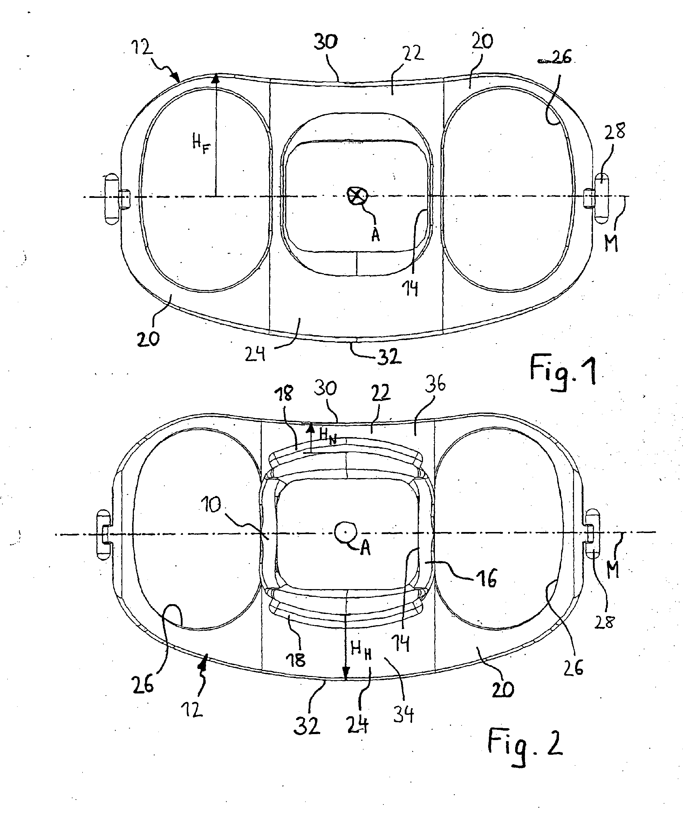

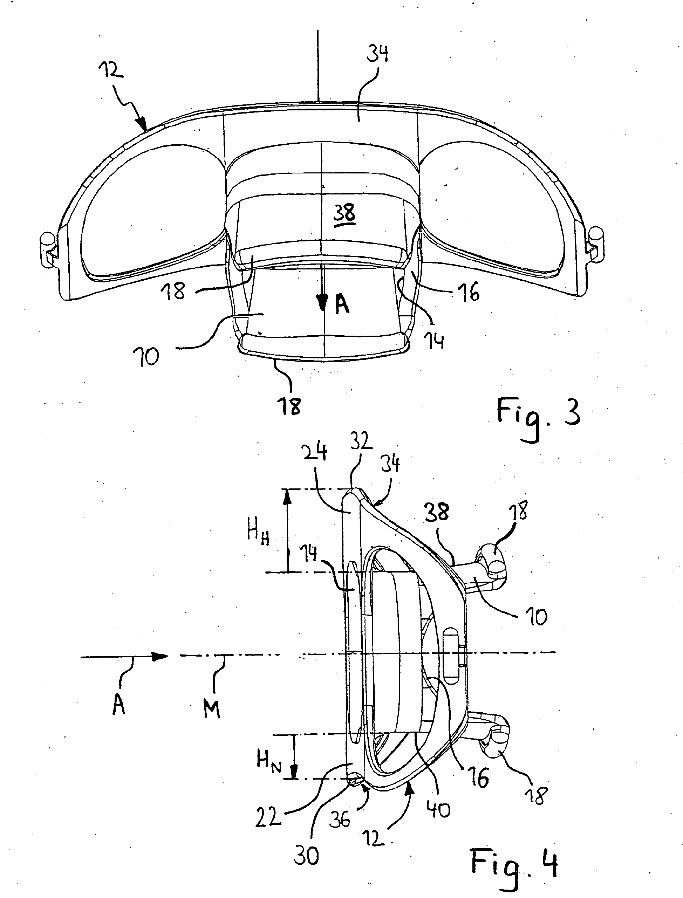

Der Beißring gemäß der in den Figuren 1 bis 4 dargestellten Ausführungsform ist als einstückiges Kunststoff-Gussteil mit einem rohrförmigen Beißtubus 10 und einem an einem Ende des Beißtubus 10 ausgebildeten Lippenschild 12 gebildet. Der Beißtubus 10 definiert eine Durchlassöffnung 14, durch welche entlang einer Mittelachse A des Beißtubus 10 ein nicht dargestelltes Instrument einführbar ist.

Für den Beißring kann eine Mittelebene M derart definiert werden, dass die Mittelebene M die Mittelachse A der Durchlassöffnung 14 aufnimmt, so dass sie den Lippenschild 12 etwa in der Mitte im Wesentlichen senkrecht schneidet. Gemäß der vorliegenden Erfindung ist dann der Beißtubus 10 bezüglich dieser Mittelebene M im Wesentlichen symmetrisch ausgebildet. Er weist im Querschnitt in etwa die Form eines Rechtecks auf, wobei in der gezeigten Ausführungsform die etwas kürzeren Rechteckseiten senkrecht zur Mittelebene M verlaufen. Eine solche Querschnittsform der Durchlassöffnung 14 kann der Form eines bestimmten Instruments angepasst sein und ist dementsprechend nicht auf die gezeigte Ausführungsform beschränkt.

An den im Wesentlichen senkrecht zur Mittelebene M verlaufenden Seitenwandungen des Beißtubus 10 weist dieser an seinem dem Lippenschild 12 abgewandten Ende jeweils eine halbkreisförmige Ausnehmung 16 auf, um in an sich bekannter Weise Eingriffsausschnitte für die Finger des medizinischen Personals bereitzustellen. Ferner ist an den von der Mittelebene M beabstandeten Randabschnitten des von dem Lippenschild 12 beabstandeten Endes des Beißtubus 10 ein Zahnschildabschnitt 18 in Form einer nach außen vorstehenden Wulst ausgebildet.

Der Lippenschild 12 ist in etwa senkrecht zur Mittelachse A des Beißtubus 10 ausgerichtet und ähnelt einer länglichen Platte, deren Längsrichtung parallel zur Mittelebene M orientiert ist und die eine Biegung um eine die Mittelachse A schneidende und senkrecht zur Mittelebene M verlaufende Achse aufweist.

Der Lippenschild 12 kann unterteilt werden in zwei Flügelabschnitte 20, welche in Längsrichtung des Lippenschilds 12 beiderseits der Durchlassöffnung 14 angeordnet sind, sowie einen niedrigen Schildabschnitt 22 und einen hohen Schildabschnitt 24, welche in zur Mittelebene M senkrechter Richtung beiderseits der Durchlassöffnung 14 angeordnet sind. Die im Wesentlichen gleich ausgebildeten Flügelabschnitte 20 weisen jeweils eine ovale Eingriffsöffnung 26 auf, durch welche während des Eingriffs ein Finger des medizinischen Personals zur Positionierung des Beißrings oder des eingeführten Instruments oder auch ein weiteres Instrument, beispielsweise eine Absaugvorrichtung, einführbar ist. An den äußersten Enden der Flügelabschnitte 20 sind ferner Einhängeabschnitte 28 vorgesehen, an denen ein um den Kopf des Patienten geführtes Halteband, insbesondere ein später noch genauer beschriebenes erfindungsgemäßes Halteband, einhängbar ist, um den Beißring während des Eingriffs in Position zu halten.

Gemäß der vorliegenden Erfindung sind der niedrige Schildabschnitt 22 und der hohe Schildabschnitt 24 so ausgebildet, dass die Höhe H_{N} des äußeren Rands 30 des niedrigen Schildabschnitts 22 geringer ist als die Höhe H_{H} des äußeren Rands 32 des hohen Schildabschnitts 24. Genauer weist der äußere Rand 30 des niedrigen Schildbereichs 22 eine konvexe Form auf, so dass die Höhe des Rands 30 über dem Beißtubus 10 am Schildbereich 22 ein Minimum hat und zu den Flügelbereichen 20 des Lippenschilds hin zunimmt. Andererseits weist der äußere Rand 32 des hohen Lippenschildbereichs 24 bezüglich der Mittelebene M eine konkave Form auf, so dass die Höhe des Rands 32 über dem Beißtubus am Schildbereich 24 ein Maximum hat und zu den Flügelbereichen 26 hin abnimmt.

Die Anwendung des Beißrings gemäß der in den Figuren 1 bis 4 dargestellten Ausführungsform der Erfindung wird im Folgenden für den Einsatz bei einem Patienten mit natürlichem Gebiss bzw. mit während des Eingriffs nicht entferntem künstlichen Gebiss beschrieben. Der Beißring wird in Richtung des Pfeils A in den geöffneten Mund des Patienten eingeführt, bis der Beißtubus 10 im Wesentlichen vollständig im Mund des Patienten aufgenommen ist und der Lippenschild 12 von außen an den Lippen in etwa anliegt. Bezüglich der Mittelachse A des Beißtubus 10 ist der Beißring dabei so gedreht, dass der hohe Schildabschnitt 24 nahe der Nase der Patienten angeordnet ist und der niedrige Schildabschnitt 22 in Richtung des Kinns des Patienten weist. Ein zwischen einer Innenseite 34 des hohen Schildbereichs 24 und dem Zahnschildabschnitt 18 verlaufender Abschnitt 38 der Außenwandung des Beißtubus 10 dient dabei als Beißfläche 38 für die oberen Schneidezähne des Patienten und ein zwischen einer Innenseite 36 des niedrigen Schildbereichs 22 und dem Zahnschildabschnitt 18 verlaufender Abschnitt 40 der Außenwandung des Beißrings 10 dient als

Beißfläche 40 für die unteren Schneidezähne des Patienten. Durch diese Beißflächen 38, 40 werden die Zähne des Patienten zurückgehalten, um eine Beschädigung oder Blockierung des einzuführenden Instruments zu verhindern. Durch den Zahnschildabschnitt 18, welcher bezüglich der Mittelebene M von den Beißflächen 38, 40 jeweils nach außen vorsteht, kann zudem vermieden werden, dass der Patient den Beißring während des Eingriffs freiwillig oder unfreiwillig ausschiebt oder der Beißring beispielsweise beim Zurückziehen des Instruments mit herausgezogen wird.

Andererseits wird die Bewegung des Beißrings in Richtung des Pfeils A dadurch begrenzt, dass der Lippenschild 12 auf den Lippen des Patienten anliegt, wobei auch die Unterlippe des Patienten, an welcher der niedrige Schildbereich 22 anliegt, durch die Unterstützung der Flügelbereiche 20 ausreichend gut zurückgehalten wird. Die Höhe H_{H} des hohen Schildbereichs 24 ist dabei so bemessen, dass bei dem Patienten mit Gebiss der hohe Schildbereich 24 die Oberlippe des Patienten einerseits ausreichend abdeckt, andererseits jedoch unterhalb der Nase endet, um ein unangenehmes Drücken oder Scheuern an der Unterseite der Nase zuverlässig zu vermeiden. Im Speziellen beträgt die Höhe H_{H} in dieser Ausführungsform der Erfindung etwa 17 mm.

Der erfindungsgemäße Beißring kann wahlweise auch bei einem Patienten verwendet werden, welcher zum Zeitpunkt des Eingriffs kein Gebiss trägt. Dazu wird der Beißring bezüglich der Mittelachse A des Beißtubus 10 so ausgerichtet, dass der niedrige Schildbereich 22 zur Nase des Patienten hin und der hohe Schildbereich 24 zum Kinn des Patienten hin ausgerichtet sind. Nach dem Einführen des Beißtubus 10 in den geöffneten Mund des Patienten liegt dann der niedrige Schildbereich 22 an der Oberlippe und der hohe Schildbereich 24 an der Unterlippe des Patienten an.

Da bei dem Patienten ohne Gebiss Ober- bzw. Unterkiefer direkt auf den Beißflächen 40 bzw. 38 aufliegen, ist bei einem solchen Patienten die Unterseite der Nase näher an der Mittelebene M positioniert als bei einem Patienten mit Gebiss. Der bei einem Patienten ohne Gebiss an der Oberlippe anliegende niedrige Schildbereich 22 weist demnach gemäß der Erfindung eine Höhe H_{N} auf, welche auch bei dieser Patientengruppe einerseits eine zuverlässige Abdeckung der Oberlippe ermöglicht und andererseits den niedrigen Schildabschnitt 22 unterhalb der Nase enden lässt. Ein zuverlässiges Zurückhalten der Oberlippe wird dabei auch durch die Flügelabschnitte 20 unterstützt. Damit ist gewährleistet, dass auch bei Patienten, die während des Eingriffs zumindest im Bereich der oberen Schneidezähne keine Zahnprothesen tragen, der Lippenschild 12 des Beißrings nicht an der Nase drückt oder scheuert. Im Speziellen beträgt die Höhe H_{N} in dieser Ausführungsform der Erfindung etwa 9 mm.

Die vorliegende Erfindung ist nicht auf die in den Figuren 1 bis 4 dargestellte Ausführungsform beschränkt. So ist es beispielsweise möglich, Größe und Querschnittsform der Durchlassöffnung 14 zu verändern und gegebenenfalls an einen bestimmten Verwendungszweck anzupassen. Ferner können Abschnitte der Beißflächen 38, 40 zum Lippenschild 12 hin stärker geneigt sein. Die Höhen H_{H} und H_{N} sollten dann von dem Bereich der jeweiligen Basisfläche aus gemessen werden, an welchem die Zähne bzw. der Oberkiefer bei normalem Gebrauch anliegen.

Auch ist es möglich, die Höhen H_{H} und H_{N} des hohen bzw. niedrigen Schildbereichs 24, 22 nicht gemäß der Anatomie von Patienten ohne bzw. mit Gebiss anzupassen, sondern diese so zu wählen, dass der Beißring für andere Patientengruppen oder Anwendungsgebiete angepasst ist.

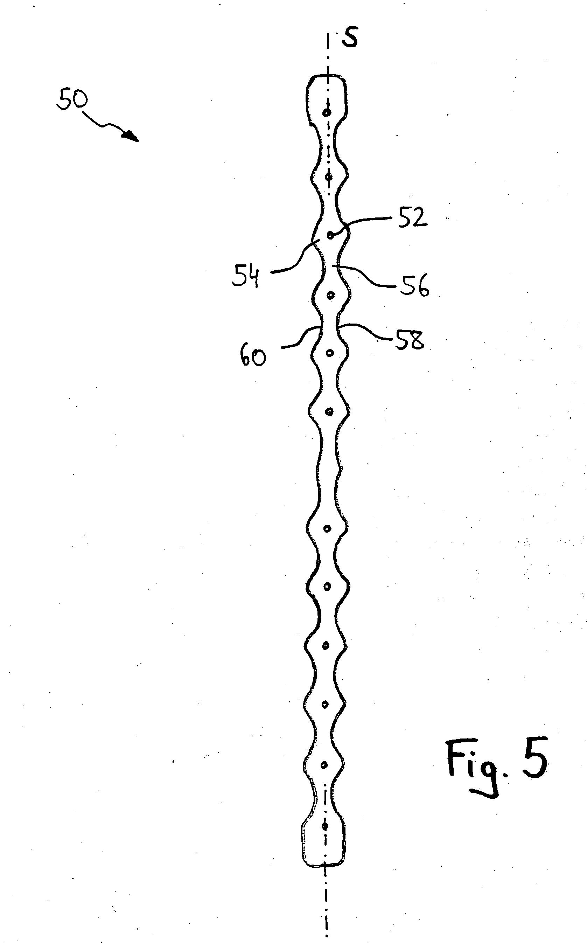

Um einen Beißring, insbesondere den in den Fig. 1 bis 4 beschriebenen Beißring, sicher am Kopf des Patienten zu halten, kann ein Halteband verwendet werden, welches den Kopf des Patienten umläuft und an den Einhängeabschnitten 28 des Beißrings eingehängt wird. In Fig. 5 ist ein Halteband gemäß einem Ausführungsbeispiel der Erfindung in einem nicht an einem Beißring montierten, in die Zeichenebene aufgerollten Zustand gezeigt. Es weist eine Mehrzahl von in Längsrichtung des Haltebandes verteilt angeordneten Löchern 52 auf, mit welchen das Halteband 50 an den Einhängeabschnitten 28 des Beißrings einhängbar ist. Durch die Wahl verschiedener der Löcher 52 kann die Länge des Haltebandes 50 auf die Größe des Kopfes des Patienten angepasst werden.

Die Löcher 52 definieren entlang des Haltebands 50 Anbringungsabschnitte 54 in der Umgebung der Löcher 52 und Zwischenabschnitte 56, welche zwischen den Anbringungsabschnitten 54 angeordnet sind. Das erfindungsgemäße Halteband 50 ist so gestaltet, dass die quer zur Längsrichtung des Haltebands 50 gemessene Breite der Anbringungsabschnitte 54 größer ist als die Breite der Zwischenabschnitte 56. Realisiert wird dies dadurch, dass die Längsränder 58, 60 des Haltebandes in einer der Zeichenebene entsprechenden Hauptebene des Haltebandes 50 derart wellenförmig verlaufen, dass das Halteband 50 bezüglich einer bei S in Fig. 5 angedeuteten Symmetrieachse symmetrisch ist. Von der Symmetrieachse S aus betrachtet definieren dann zwei gegenüberliegende Wellenberge der Ränder 58, 60 jeweils einen Anbringungsabschnitt 54, während die Zwischenabschnitte 56 zwischen zwei gegenüberliegenden Wellentälern der Seitenränder 58, 60 definiert sind. Durch eine solche Struktur liegt entlang des Haltebands 50 an allen Stellen im Wesentlichen die gleiche Reißfestigkeit vor.

In dem in Fig. 5 gezeigten Ausführungsbeispiel kann die Dicke des Haltebandes 50 über seine gesamte Länge im Wesentlichen konstant gehalten werden, was sich vorteilhaft auf das Tragegefühl am Kopf des Patienten auswirken kann. Es ist jedoch ebenso möglich, Breite und Dicke des Haltebandes in den Anbringungsabschnitten größer zu gestalten als in den Zwischenabschnitten. In einem weiteren Ausführungsbeispiel kann die Breite des Haltebandes über seine gesamte Länge im Wesentlichen konstant sein, während die Dicke des Haltebandes in den Anbringungsabschnitten größer ist als in den verbleibenden Abschnitten, insbesondere den Zwischenabschnitten des Haltebandes. Auch mit einer solchen Struktur kann entlang des Haltebandes an allen Stellen im Wesentlichen die gleiche Reißfestigkeit bereitgestellt werden und der Material- und Kostenaufwand bei der Herstellung minimiert werden.

## Patentansprüche

1. Beißring mit einem eine Durchlassöffnung (14) zum Einführen eines Instruments in den Mund einer Person umschließenden Beißtubus (10) und einem von auf sich gegenüberliegenden Seiten einer Mittelebene (M) des Beißtubus (10) von diesem quer zur Mittelebene (M) abstehenden Lippenschild (12), wobei der Beißtubus (10) bezogen auf die Mittelebene (M) im Wesentlichen symmetrisch ausgebildet ist, **dadurch gekennzeichnet, dass**
der Lippenschild (12) zumindest an seinem mittig zum Beißtubus (10) gelegenen Bereich (22, 24) auf einer Seite der Mittelebene niedriger ist als auf seiner gegenüberliegenden Seite, und dass die Höhe (H_{N}) des niedrigeren Bereichs (22) des Lippenschilds (12) über einer zugewandten Beißfläche (40) des Beißtubus (10) zwischen 2 und 15 mm liegt und die Höhe (H_{H}) des höheren Bereichs (24) des Lippenschilds (12) über einer zugewandten Beißfläche (38) des Beißtubus (10) zwischen 10 und 30 mm liegt.

2. Beißring nach Anspruch 1, **dadurch gekennzeichnet, dass** bezüglich der Mittelebene (M) ein äußerer Rand (30) des niedrigeren Lippenschildbereichs (22) eine konkave Form aufweist und ein äußerer Rand (32) des höheren Lippenschildbereichs (24) eine konvexe Form aufweist.

3. Beißring nach einem der vorhergehenden Ansprüche, **dadurch gekennzeichnet, dass** die Höhe (H_{N}) des niedrigeren Lippenschildbereichs (22) zwischen 6 und 12 mm liegt.

4. Beißring nach einem der vorhergehenden Ansprüche, **dadurch gekennzeichnet, dass** die Höhe (H_{H}) des höheren Lippenschildbereichs (24) zwischen 15 und 25 mm liegt.

5. Beißring nach einem der vorhergehenden Ansprüche, **dadurch gekennzeichnet, dass** die Durchlassöffnung (14) des Beißtubus (10) zumindest im Bereich des Lippenschilds (12) im Wesentlichen die Querschnittsform eines Rechtecks, dessen längere Seiten parallel zur Mittelebene (M) liegen, aufweist.

6. Beißring nach einem der vorhergehenden Ansprüche, **dadurch gekennzeichnet, dass** er ein an diesem angebrachtes oder anbringbares oder ausgebildetes Halteband (50) aufweist, wobei entlang des Haltebandes (50) eine Mehrzahl von durch Löcher (52) oder Ausnehmungen in dem Halteband (50) gebildete Anbringungsabschnitte (54) angeordnet sind, welche an mindestens einem Einhängeabschnitt (28) des Beißrings einhängbar sind, und
wobei die Breite oder/und die Dicke des Haltebandes (50) in den Anbringungsabschnitten (54) größer ist als in den Anbringungsabschnitten (54) benachbarten Abschnitten (56) des Haltebandes (50).

7. Beißring nach Anspruch 6, **dadurch gekennzeichnet, dass** die Breite oder/und die Dicke des Haltebandes (50) in den Anbringungsabschnitten (54) und die Breite oder/und die Dicke des Haltebandes (50) in den den Anbringungsabschnitten (54) benachbarten Abschnitten (56) des Halte bandes (50) so gewählt sind, dass für eine Zugbeanspruchung entlang der Längsrichtung (S) des Haltebandes (50) eine Reißfestigkeit des Haltebandes (50) in allen in der Längsrichtung aufeinanderfolgenden Abschnitten (54, 56) des Haltebandes (50) im Wesentlichen groß ist.

8. Beißring nach Anspruch 6 oder Anspruch 7, **dadurch gekennzeichnet, dass** die beiden Längsränder (58, 60) des Haltebandes (50) zumindest abschnittsweise in einer Hauptebene des Haltebandes (50) derart wellenförmig verlaufen, dass Abschnitte (54) mit größerer Breite und Abschnitte (56) mit kleinerer Breite abwechselnd ineinander übergehen, wobei die Anbringungsabschnitte (54) in den Abschnitten größerer Breite vorgesehen sind.

## Claims

1. Bite block comprising a bite tube (10) which surrounds a through-opening (14) for introducing an instrument into the mouth of a person, and comprising a lip shield (12) which protrudes from opposing sides of a central plane (M) of the bite tube (10) and therefrom transversely to the central plane, the bite tube (10) being substantially symmetrical relative to the central plane (M), **characterised in that** the lip shield (12), at least in the region (22, 24) thereof which is central relative to the bite tube (10), is lower on one side of the central plane than on the opposite side thereof, and **in that** the height (H_{N}) of the lower region (22) of the lip shield (12) is between 2 and 15 mm above a facing bite surface (40) of the bite tube (10), and the height (H_{H}) of the higher region (24) of the lip shield (12) is between 10 and 30 mm above a facing bite surface (38) of the bite tube (10).

2. Bite block according to claim 1, **characterised in that**, relative to the central plane (M), an outer edge (30) of the lower lip-shield region (22) is concave and an outer edge (32) of the higher lip-shield region (24) is convex.

3. Bite block according to any of the preceding claims, **characterised in that** the height (H_{N}) of the lower lip-shield region (22) is between 6 and 12 mm.

4. Bite block according to any of the preceding claims, **characterised in that** the height (H_{H}) of the higher lip-shield region (24) is between 15 and 25 mm.

5. Bite block according to any of the preceding claims, **characterised in that** the cross section of the through-opening (14) in the bite tube (10), at least in the region of the lip shield (12), is substantially rectangular, the longer sides of which are parallel to the central plane (M).

6. Bite block according to any of the preceding claims, **characterised in that** it comprises a retaining band (50) which is or can be attached thereto or formed thereon, a plurality of attachment portions (54) formed by holes (52) or recesses in the retaining band (50) being arranged along the retaining band (50), which attachment portions can be hooked onto at least one hooking-on portion (28) of the bite block, and the width and/or thickness of the retaining band (50) being greater in the attachment portions (54) than in the portions (56) of the retaining band (50) adjacent to the attachment portions (54).

7. Bite block according to claim 6, **characterised in that** the width and/or thickness of the retaining band (50) in the attachment portions (54) and the width and/or thickness of the retaining band (50) in the portions (56) of the retaining band (50) adjacent to the attachment portions (54) are selected such that in the case of a tensile load in the longitudinal direction (S) of the retaining band (50), a tear resistance of the retaining band (50) is substantially high in all the portions (54, 56) of the retaining band (50) which are consecutive in the longitudinal direction.

8. Bite block according to either claim 6 or claim 7, **characterised in that** the two longitudinal edges (58, 60) of the retaining band (50) extend in an undulating manner, at least in portions in a main plane of the retaining band (50), such that portions (54) having a greater width and portions (56) having a smaller width transition into one another alternately, the attachment portions (54) being provided in the portions having a greater width.

## Revendications

1. Anneau de protection dentaire, comportant un tube de protection dentaire (10), entourant une ouverture de passage (14) permettant l'introduction d'un instrument dans la bouche d'une personne, et une plaque labiale (12) s'écartant sur des côtés en vis-à-vis d'un plan médian (M) du tube de protection dentaire (10), à partir de celui-ci transversalement au plan médian (M), le tube de protection dentaire (10) étant réalisé sensiblement symétriquement par rapport au plan médian (M),
**caractérisé en ce que**
la plaque labiale (12), au moins dans sa zone (22, 24) située au centre par rapport au tube de protection dentaire (10), est plus petite sur un côté du plan médian que sur son côté opposé, et **en ce que** la hauteur (H_{N}) de la zone plus petite (22) de la plaque labiale (12) mesure entre 2 et 15 mm depuis une surface de serrement des dents (40) du tube de protection dentaire (10) orientée vers ladite zone, et la hauteur (H_{H}) de la zone plus grande (24) de la plaque labiale (12) mesure entre 10 et 30 mm depuis une surface de serrement des dents (38) du tube de protection dentaire (10) orientée vers ladite zone.

2. Anneau de protection dentaire selon la revendication 1, **caractérisé en ce que** par rapport au plan médian (M), un bord extérieur (30) de la zone plus petite (22) de la plaque labiale présente une forme concave et un bord extérieur (32) de la zone plus grande (24) de la plaque labiale présente une forme convexe.

3. Anneau de protection dentaire selon l'une quelconque des revendications précédentes, **caractérisé en ce que** la hauteur (H_{N}) de la zone plus petite (22) de la plaque labiale mesure entre 6 et 12 mm.

4. Anneau de protection dentaire selon l'une quelconque des revendications précédentes, **caractérisé en ce que** la hauteur (H_{H}) de la zone plus grande (24) de la plaque labiale mesure entre 15 et 25 mm.

5. Anneau de protection dentaire selon l'une quelconque des revendications précédentes, **caractérisé en ce que** l'ouverture de passage (14) du tube de protection dentaire (10), au moins dans la zone de la plaque labiale (12), comporte une section transversale ayant sensiblement la forme d'un rectangle, dont les grands côtés sont parallèles au plan médian (M).

6. Anneau de protection dentaire selon l'une quelconque des revendications précédentes, **caractérisé en ce qu'**il comporte une bande de maintien (50) attachée ou pouvant être attachée ou réalisée sur celui-ci, une pluralité de tronçons d'attache (54), formés par des trous (52) ou des évidements dans la bande de maintien (50) étant disposés le long de la bande de maintien (50), lesquels tronçons d'attache peuvent être accrochés à au moins un tronçon d'accrochage (28) de l'anneau de protection dentaire, et
la largeur et/ou l'épaisseur de la bande de maintien (50) dans les tronçons d'attache (54) étant supérieure à celle de tronçons (56) de la bande de maintien (50) adjacents aux tronçons d'attache (54).

7. Anneau de protection dentaire selon la revendication 6, **caractérisé en ce que** la largeur et/ou l'épaisseur de la bande de maintien (50) dans les tronçons d'attache (54) et la largeur et/ou l'épaisseur de la bande de maintien (50) dans les tronçons (56) de la bande de maintien (50) adjacents aux tronçons d'attache (54) sont choisies de telle sorte que, pour une sollicitation de traction le long de la direction longitudinale (S) de la bande de maintien (50), une résistance à la déchirure de la bande de maintien (50) est sensiblement élevée dans tous les tronçons (54, 56) de la bande de maintien (50) successifs dans la direction longitudinale.

8. Anneau de protection dentaire selon la revendication 6 ou la revendication 7, **caractérisé en ce que** les deux bords longitudinaux (58, 60) de la bande de maintien (50) s'étendent sous forme ondulée, au moins par zones, dans un plan principal de la bande de maintien (50), de telle sorte que des tronçons (54) de plus grande largeur et des tronçons (56) de plus petite largeur se succèdent en alternance, les tronçons d'attache (54) étant prévus dans les tronçons de plus grande largeur.
